# EUROPEAN PATENT APPLICATION

(11) **EP 2 017 191 A1**
(43) Date of publication of application: **21.01.2009**
(21) Application number: 07742941.3
(22) Date of filing: 08.05.2007
(51) Int. Cl.: B65D 75/30, A23L 1/00, A61K 9/00, A61K 47/36, A61K 47/42, B65D 65/46

(54) **EDIBLE FILM PACKAGE HAVING PRODUCT FOR ORAL INTAKE ENCLOSED THEREIN**

(30) Priority: 10.05.2006 JP 2006131479
(71) Applicant: Kureha Corporation, Tokyo 103-8552 (JP)
(72) Inventor: ISHII, Kazumi, Tokyo 169-8503 (JP); ONO, Saichi, Tokyo 169-8503 (JP); YAMAGUCHI, Iwao, Toda-shi Saitama, 355-0012 (JP)
(74) Representative: HOFFMANN EITLE
(86) International application number: PCT/JP2007/059506
(87) International publication number: WO 2007/129706

(57) **Abstract**

This invention aims to provide a novel taking or having-or-swallowing form for an oral material, such as drugs or foodstuffs.

This invention provides an edible film packaging 1 containing an edible film material 10 which has adhesion properties and is softened upon contact with moisture. The edible film packaging 1 is taken or had-or-swallowed while an oral material being encapsulated. Moreover, this invention provides an oral-material taking method, including taking or having-or-swallowing the edible film packaging 1 in which the oral material 2 has been encapsulated as it is without opening or as it is without opening after being immersed in water for a given period of time.

## Description

### Technical Field

The present invention relates to an edible film packaging encapsulating an oral material. More specifically, the present invention relates to an edible film packaging encapsulating an oral material, such as oral taking drugs or quasi drugs, or foodstuffs and which is devised to take, or have-or-swallow as it is.

### Background Art

The pharmaceutical forms of oral taking drugs or quasi drugs can be roughly classifies into capsules, tablets, sa-called powdered medicines, such as powder, subtle granules, and pellets, jelly agents, liquid agents, etc. The pharmaceutical forms are suitably selected in terms of types and symptoms of diseases, types of drugs, ease of taking (hereinafter referred to as "taking properties"), etc.

With respect to the taking properties, each pharmaceutical form has advantages and disadvantages, and thus preferable pharmaceutical forms vary depending on individuals and ages. In the case of drugs whose contents have bad taste and odor or a strong stimulus, capsules have high masking properties and are easy to take. Moreover, capsules are advantageous in that their colors and sizes can also be suitably selected, which makes it easy to distinguish drugs. In contrast, capsules are disadvantageous in that, in some cases, capsules are stuck in the throat at the time of taking, or when the capsule size is large, swallowing of such capsules is difficult. Depending on the case, capsules are disadvantageous in that the drug adheres to the esophageal mucosa, causing esophagitis and esophageal ulcer.

Tablets are advantageous in that they are convenient to carry, relatively easy to adjust the absorbed parts by covering the tablet surfaces, etc. In contrast, similarly as in capsules, when the tablet size is large, swallowing of such tablets is difficult or tablets are stuck in the throat in some cases. Moreover, esophagitis and esophageal ulcer may be developed. Furthermore, tablets are disadvantageous in that it is hard to finely adjust a dose as in powdered medicine, such as powder, subtle granules, and pellets.

Powdered medicines, such as powder, subtle granules, and pellets are advantageous in that fine adjustment of a dose can be performed, and breakdown and elution of preparations generally occur promptly as compared with tablets or capsules, and thus drugs are easily absorbed into the body.

The powdered medicines, such as powder, subtle granules, and pellets, are popular pharmaceutical forms in the Asian area including Japan which has the oriental medicine culture because the origin of the powdered medicines is boiling herbal medicines. Thus, the powdered medicines are pharmaceutical forms which are relatively easy to accept at the time of taking. In contrast, in Europe and the United States in which the Western medicine culture is popular, such pharmaceutical forms do not exist, and capsules and tablets are mainly used. Thus, the powdered medicines are not accepted.

The powdered medicines, such as powder, subtle granules, and pellets are disadvantageous in that, at the time of taking, users are likely to feel the taste and odor of drugs or quasi drugs, and to feel unpleasantness caused by adherence of the powdered medicines to the mucous membrane of an oral cavity or by entrance to the clearance between false teeth, for example. Therefore, the powdered medicines tend to be rejected by particularly children and elderly persons, and thus taking assist products are used in many cases.

As the taking assist products, wafer paper, taking assist jellies, etc., are commercially available, for example. However, the taking assist products are disadvantageous in that they need to be separately purchased in addition to drugs or quasi drugs, for example. Moreover, the taking assist products cause patients to wrap drugs or quasi drugs in wafer paper or to mix taking assist jelly with drugs or quasi drugs by themselves, which are complicated for the patients. During such operation, drugs or quasi drugs may be spilt, and thus a proper dose may be unable to be taken. Furthermore, using wafer paper is also disadvantageous in that individual differences arise in how to wrap drugs or quasi drugs, and thus the drugs or quasi drugs jump out in the mouth at the time of taking depending on the wrapping manner.

Since liquids and jellies are poured in the throat, they are excellent in taking properties. Syrups to which sweet flavor is imparted are suitable for children. Moreover, in drugs or quasi drugs with foreign substance feeling, such as resin drugs which do not dissolve in water, taking properties are improved by palate texture (smoothness) of jellies in many cases. However, there is a disadvantage in that when bitterness or the like of an active ingredient itself is strong, it is difficult to suppress bitterness or the like by a sweetening agent or the like, even when the taste or the like is devised. Moreover, there is a disadvantage in that when formed into jellies, a taking volume becomes large, which deteriorates portability.

In addition, Patent Document 1 discloses a packaging for drugs formed of food materials, such as starch, protein, or a dietary fiber. Patent Document 2 discloses an edible phosphoprotein film into which mouth care agents, pharmacological or veterinary medicinal active substances, neutraceutical agents, saliva stimulants, vitamins, and minerals, have been kneaded. Moreover, Patent Document 3 discloses a dissolution packaging article which can be dissolved in hot water to release the contents in hot water. All of the packaging and the like dissolve at the time of preparation or in the mouth.

Patent Document 4 discloses an edible film packaged foodstuffs in which an edible film and an edible raw material as contents have been adhered to each other and solidified. Such edible film packaged foodstuffs refer to forms of a so-called sausage. Patent Document 5 discloses an edible strip packaging, substituting for capsules, in which liquid or powdered drugs or foodstuffs are contained. The edible strip packaging refers to a packaging which has been designed to swallow as it is, similarly as the conventional capsules.
Patent-Document 1: Japanese Unexamined Patent Application Publication No. 11-321921
Patent-Document 2: Japanese Unexamined Patent Application Publication No. 2006-506981
Patent-Document 3: Japanese Unexamined Patent Application Publication No. 06-183456
Patent Document 4: Japanese Unexamined Patent Application Publication No. 08-84565
Patent-Document 5: US2836291

### Disclosure of the Invention

### Technical Problems to be Solved

As described above, the pharmaceutical forms of drugs or quasi drugs have advantages and disadvantages as viewed in terms of only the taking properties. In view of the change in the age population structure with increase in the aging population, the change in food taste in which fluids tend to be preferred to chewing articles, the progress in the development of new drugs or quasi drugs, etc., it is expected that a novel pharmaceutical form field different from the conventional pharmaceutical form field is demanded from now on. In fact, according to the report on " Research and development on a novel packaging film and container for the optimal administration to the elderly people " (the health science group) in 1988, pharmaceutical forms excellent in taking properties as compared with the conventional pharmaceutical forms are demanded. Specifically, jelly agents, yoghurt agents, pudding agents, candy agents, biscuit agents, chewing gum agents, etc., are mentioned as pharmaceutical forms which are desired to develop in the future.

Moreover, with the background of growing concern on health, changes in lifestyle, etc., so-called functional foods have been on the rise, which forms one of the rapid-growth fields of a food market in recent years. Foodstuffs containing functional components having an optimum intake amount similarly as in drugs or quasi drugs tend to be naturally provided in the same pharmaceutical forms of drugs or quasi drugs or similar product forms (e.g., a powder form, a tablet form, a capsule form, etc.). It is expected that having-or- swallowing forms thereof are also diversified from now on with diversification of foodstuffs represented by the functional foods beyond the taking forms of drugs or quasi drugs.

Thus, an object of the present invention is to provide a packaging form having a novel taking form or having-or-swallowing form concerning oral materials, such as oral taking drugs or quasi drugs, or foodstuffs. More specifically, a primary object of the present invention is to provide a novel packaging encapsulating oral materials, such as oral taking drugs or quasi drugs, or foodstuffs and which can be taken or had-or-swallowed as it is, and a novel oral intake method using the packaging.

### Means to Solve the Problems

In order to achieve the above-described objects, the present inventors have studied on various packaging forms. As a result, the present inventors found a new packaging form which allows more excellent oral intake than the conventional intake form or having-or-swallowing form.

First, the present invention provides an edible film packaging containing an edible film material which has adhesion properties and is softened upon contact with moisture. The packaging is an edible film packaging which can be taken or had-or-swallowed as it is while an oral material being encapsulated.

Here, "encapsulating an oral material" refers to a packaging form which prevents an oral material wrapped with a wrapping material in a dry state from jumping out of the packaging. There is no limitation on the form or size of the packaging.
The "adhesion properties" refer to properties with which facing edible film materials do not separate due to a certain phenomenon, and the adhesion principle is not narrowly limited. As an example, materials having thermal adhesion properties (heat sealing properties) are more preferable from the viewpoint of package processability.

The thickness of the edible film material forming the edible film packaging is preferably from 20 to 50 µm. This thickness range refers to a thickness in which the edible film does not dissolve, and is merely softened in the mouth, i.e., thickness excellent in ease of taking or having-or-swallowing, and a thickness in which the edible film dissolves to promptly release the contents in the stomach or intestines after taking or having- or - swallowing, i.e., preferable thickness in terms of digestive absorptivity.

Moreover, when the edible film material forming the edible film packaging is placed on an opening part of a 10 mL beaker specified in Japanese Industrial Standard (JIS standard) R3503; the edible film material is fixed with a rubber band in such a manner as to cover the opening part of the beaker; then a 1 g weight specified in Japanese Industrial Standard (JIS standard) B7609 is put on the center portion of the edible film material; and 50 µL of 25°C purified water is added to a depressed part of the edible film material due to the 1g weight, it is preferable that a period of time from the edible film material is broken until the 1 g weight is dropped in the 10 mL beaker is 2 minutes or more and lower than 10 minutes.

This method refers to a method of measuring the strength against water of the edible film material forming the edible film packaging according to the present invention. When the drop time is longer, the strength against water is higher. An edible film material having the drop time measured by the method of 2 minutes or more and lower than 10 minutes has both ease of taking and having-or-swallowing with which the edible film does not dissolve, and is merely softened in the mouth and digestive absorptivity with which the edible film promptly dissolves to release the contents in the stomach or intestines after taking or having-or-swallowing.

Various materials can be selected as the oral materials forming the edible film packaging insofar as they are oral materials, such as oral taking drugs or quasi drugs, or foodstuffs.

The present invention further provides a method of taking an oral material including taking or having-or-swallowing the edible film packaging as it is without opening, and a method of taking an oral material including immersing the edible film packaging in water for a given period of time, and then taking or having-or- swallowing the edible film packaging as it is without opening.

Hereinafter, the technical terms used in the present invention are defined. In the present invention, the "edible film material" refers to a packaging film material which has properties suitable for having-or-swallowing, more specifically, which is safe to the living body when had-or-swallowed, and which contains, as a constitutional element, a raw material, such as a natural polymer, which can release an encapsulated oral material in a given period of time irrespective of digestibility and indigestibility. The "softening" means that hard physical properties are changed to soft physical properties.

### Effects of the Invention

The present invention designs an edible film material which is softened with water or saliva when taken with water or put in the mouth as it is, and is easily deformed, and employs the same. Thus, even when an edible film packaging encapsulating an oral material, such as oral taking drugs or quasi drugs, or foodstuffs, is taken as it is, there is no unpleasantness feeling due to smooth taking feeling and palate texture, and thus problems that it is stuck in the throat or esophagus do not arise.

Moreover, due to the adhesion properties and strength against water of the edible film material, a state where an oral material has been encapsulated in the edible film material is maintained until the edible film material reaches the stomach or intestines. That is, the edible film packaging is not broken at the time of taking or having-or-swallowing, and thus the oral material does not jump out into the mouth or esophagus.

Therefore, the edible film packaging of the present invention can provide a completely new pharmaceutical form which has both the properties of ease of taking or ease of having- or- swallowing and digestive absorptivity in the living body.

### Best Mode for Carrying Out the Invention

Hereinafter, preferable embodiment for carrying out the present invention will be described with reference to the drawings. It should be noted that the embodiments described below describe one example of the typical embodiment of the present invention, and the scope of the present invention is not limited to the following embodiments.

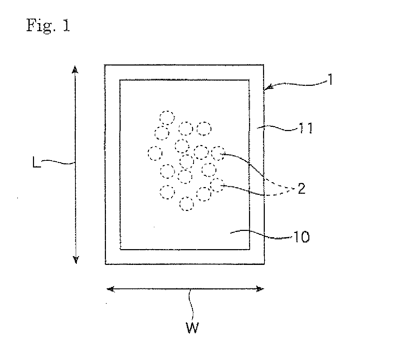

Fig. 1 is a view illustrating one embodiment of an edible film packaging 1 according to the present invention.

The edible film packaging 1 shown in Fig. 1 contains a 30 µm thick edible film material 10 and has a so-called four-side sealed packaging form in which the four sides have been sealed. The packaging size is adjusted to, for example, a width W of 20 mm and a length L of 30 mm. It should be noted that the packaging form is not limited to the four-side sealed form, and may be a three-side sealed form, a pillow packaging form, etc. The packaging size is not limited to the above-mentioned size either, and can be suitably designed according to purposes and attributes, such as user's age. Moreover, the packaging shape is not limited to an example shown in Fig. 1, and can be suitably designed.

In the edible film packaging 1, a given amount (given number) of an oral material 2 is encapsulated by a sealed part 11. For example, granular (grain-like) oral taking drugs or quasi drugs, foodstuffs, etc., as shown in Fig. 1 are encapsulated. A user puts the edible film packaging 1 in the mouth without opening, and then takes or has-or-swallows the edible film packaging 1 while softening it with saliva or after softening it by immersing it in water. The edible film packaging 1 does not dissolve with saliva or a slight amount of water, and is merely softened. Therefore, the encapsulated oral material 2 does not jump out of the edible film packaging 1 through the mouth, throat, and esophagus, and then reaches the stomach or intestines. It should be noted that the form of the oral material 2 is not limited to granular shape (grain shape) as shown in Fig. 1, and may be any forms, such as powder or tablets, except liquid articles containing moisture.

The reason why a liquid article containing moisture cannot be encapsulated in the edible film packaging 1 according to the present invention is as follows. The edible film packaging 1 according to the present invention contains an edible film material 11 which is softened with saliva or moisture. Therefore, the edible film packaging 1 according to the present invention is easily softened with a small amount of moisture, such as saliva while requiring neither hot water higher than a certain temperature nor a large amount of water. Consequently, when a liquid article containing moisture is encapsulated, the edible film packaging 1 is softened other than at the time of taking or having-or-swallowing. Moreover, with respect to the edible film packaging 1 according to the present invention, the edible film dissolves after a given period of time after contact with moisture, and then the encapsulated oral material is released. Accordingly, encapsulating a liquid article containing moisture produces a possibility that the oral material may be released other than at the time of taking or having-or-swallowing.

Raw materials and thickness of the edible film material 10, multilayering the edible film material 10, etc., can be designed at will. Hereinafter, a method of forming the edible film material 10 and the edible film packaging 1, and a method of taking or having-or-swallowing the oral material 2 to be encapsulated and the edible film packaging 1 will be described in detail.

The edible film material 10 forming- the edible film packaging 1 according to the present invention is a packaging material used for encapsulating the oral material 2 typified by oral taking drugs or quasi drugs, foodstuffs, etc., and contains a raw material causing no problems at the time of taking or having-or-swallowing the edible film packaging 1 encapsulating the oral material 2 as it is. Moreover, the edible film material 10 has adhesion properties so that the oral material 2 can be encapsulated, and has properties that it is softened upon contact with water.

It is preferable to form the edible film material 10 with a natural polymer raw material having the above-described properties. Examples include natural protein, such as casein (milk protein), gelatin, collagen, and soybean protein; natural polysaccharides, such as starch, cornstarch, dextrin, alginic acid, pullulan, pectin, cellulose, seaweed polysaccharides, such as carrageenan and agar, and soybean polysaccharides; natural dietary fibers, such as chitin and chitosan; and a combination of the above raw materials.

The above-mentioned natural polymer raw materials may be used singly, or a raw material containing two or more materials according to the purpose may be used. Moreover, components, such as pharmaceutical additives and food additives, and components having specific effects may be mixed. For example, by adding vitamin C, E, or the like, the edible film material 10 can be imparted with anti-oxidant function. Moreover, by adding a digestive organ mucosa protective ingredient, stimulus to the digestive organ mucosa by the encapsulated oral material 2 can be suppressed. Furthermore, by adding an ingredient having an effect of promoting absorption, the absorption of the encapsulated oral material 2 can be promoted. For example, by adding vitamin C to the edible film material 10 at the time of encapsulating an iron preparation, the absorption of the iron preparation is promoted.

Moreover, in order to increase ease of taking or ease of having-or-dinking of the edible film packaging 1 and the digestive absorptivity of the oral material 2, it is also possible to add glycerol and sugar alcohols for the purpose of adjusting the flexibility and strength of the edible film.

Furthermore, flavor or color may be added to a natural polymer raw material to further increase ease of taking or ease of having-or-swallowing. In addition, printing of a character, an image, etc., indicating the oral material 2 to be encapsulated can be added so that a method of taking or having-or-swallowing the edible film packaging 1, the designation of the oral material 2, the identification code, etc., can be recognized from the outside.
It is possible to describe information on items requiring attention at the time of taking or having-or-swallowing the edible film packaging 1. Furthermore, it is also possible to give information on qualities of the edible film packaging 1, such as a manufacturing-control number, an expiration date, and a use-by date.

The thickness of the edible film material 10 can be suitably designed according to the formation of the edible film material 10. When the edible film material 10 is excessively thin, the edible film material 10 is likely to break upon contact with moisture, saliva, etc. In such a case, a possibility that the encapsulated oral material 2 jumps out of the edible film packaging 1 before taking or having-or-swallowing or in the mouth becomes high, which makes it difficult to take or have-or-swallow. On the contrary, when the edible film material 10 is excessively thick, the edible film material 10 is hard to soften upon contact with moisture, saliva, etc., and does not deform in the mouth, resulting in difficulty of swallowing. Furthermore, when the edible film material 10 is excessively thick, the edible film packaging 1 itself does not easily dissolve in the stomach or intestines after taking or having-or-swallowing, and thus the encapsulated oral material 2 is difficult to release out of the edible film packaging 1, resulting in deteriorated digestive absorptivity. Accordingly, a suitable thickness varies according to the type of the edible film material.

When a common edible film material 10, such as, a starch film, a gelatin film, a casein film, a pullulan film, and an alginic acid film, is used for the edible film packaging 1 according to the present invention, the thickness thereof is preferably 20 to 50 µm. With respect to a suitable thickness of various edible films, for example, a gelatin film has a thickness of preferably lower than 40 µm, and a casein film has a thickness of preferably lower than 80 µm and more preferably lower than 50 µm. This thickness range refers to a thickness in which the edible film does not dissolve, and is merely softened in the mouth, i.e., thickness excellent in ease of taking or having- or- swallowing, and a thickness in which the edible film promptly dissolves to release the contents in the stomach or intestines after taking or having-or-swallowing, i.e., preferable thickness in terms of digestive absorptivity.

Moreover, it is preferable for the edible film material 10 to have moderate strength against water. This is because, in the case where the edible film material 10 is easily broken upon contact with water, the surface of the edible film packaging 1 is broken in the mouth, and thus the encapsulated oral material 2 jumps out thereof in the mouth or the like, resulting in reduced taking properties. Moreover, on the contrary, when the strength against water is excessively high, there may arise a risk that the encapsulated oral material 2 is not easily released from the edible film packaging 1 after taking the edible film packaging 1, resulting in reduced digestive absorption of the oral material 2.

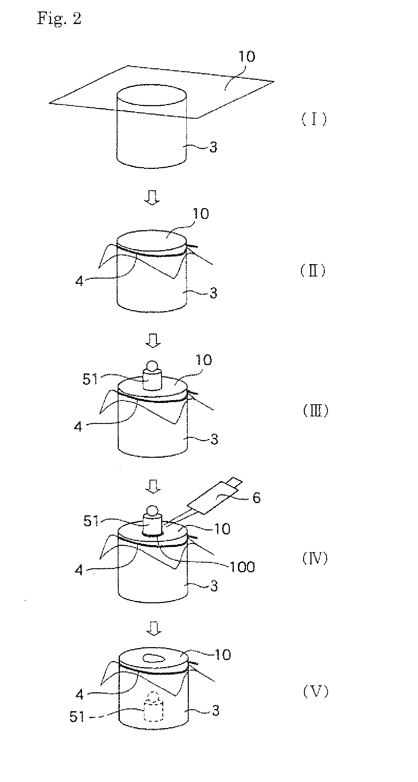

An example of a method of measuring the strength against water of the edible film material 10 will be described with reference to Fig. 2. The edible film material 10 is placed on an opening part of a 10 mL beaker 3 specified in Japanese Industrial Standard (JIS standard) R3503 (see Fig.2(1)), and then the edible film material 10 is fixed with a rubber band 4 in such a manner as to cover the opening part of the beaker 3 (see Fig. 2(II)). Then, a 1 g weight 51 specified in Japanese Industrial Standard (JIS standard) B7609 is put on the center portion of the edible film material 10 (see Fig. 2(III)); and 50 µL of 25°C purified water is added using a micropipet 6 to a depressed part 100 of the edible film material 10 due to the weight 51 (see Fig. 2(IV)). Then, a period of time from the edible film material 10 is broken until the weight 51 is dropped in the beaker 3 is measured (see Fig. 2(V)).

This method is a method of measuring the drop time of the 1 g weight 51 as a parameter representing the strength against water. When the drop time is longer, the strength against water is higher. When the drop time is shorter, the strength against water is lower. It is preferable for the edible film material 10 forming the edible film packaging 1 according to the present invention to have the drop time measured by the method of 2 minutes or more and lower than 10 minutes. This is in order to achieve both the properties of ease of taking and having-or-swallowing with which the edible film does not dissolve and is merely softened in the mouth, and digestive absorptivity with which the edible film promptly dissolves to release the contents in the stomach or intestines after taking or having-or-swallowing.

As described above, the edible film material 10 is formed using raw materials according to the purpose, designed into a thickness and width according to the purpose, and then subjected to packaging processing for encapsulating the oral material 2. In this packaging processing, a given amount of the target oral material 2 is encapsulated. For example, in this package processing, utilizing the adhesion properties of the edible film material 10, the packaging form, such as a three-side sealed form or a four-side sealed form, is suitably selected according to the purpose, and then a required unit amount of the oral material 2 is encapsulated to give the edible film packaging 1. The packaging forms, such as sealing forms, or the size or form of the edible film packaging 1 can be designed in such a manner as to be suitable for taking or having-or-swallowing, and thus are not limited to a specific form and the like.

Mentioned as an adhesion method are, for example, an adhesion method utilizing heat (a so-called heat seal), an adhesion method utilizing moisture, a method using, as an adhesive, a material to which the edible film material 10 has been dissolved, etc. The adhesion method is not limited, and adhesion can be affected by various methods. Heat sealing which has been spread as an adhesion technique is preferable in terms of industrial production.

The oral material 2 as the contents of the edible film packaging 1 is not limited. For example, oral taking drugs or quasi drugs, foodstuffs, etc., in the form of dry powder, granule, and grain are preferable. Moreover, pills, tablets, capsules, etc., can also be encapsulated.

The oral material 2 is not limited to the above case where a single kind of the oral material 2 is encapsulated. It may be freely devised in such a manner that several kinds of oral taking drugs or quasi drugs, foodstuffs, etc., are encapsulated together so as to take or have-or-swallow them at once. For example, by encapsulating several kinds of vitamin tablets together, the edible film packaging 1 which can be taken as a multivitamin can be obtained. Moreover, by encapsulating a target oral taking drug and the like and an oral taking drug having an effect of reducing the side effect of the target oral taking drug and the like together, the development of the side effect can also be suppressed. For example, a painkiller and the like which are likely to apply a load to a digestive organ and a digestive organ mucosa protective agent and the like can be encapsulated together, or a diuretic and the like which are likely to cause hypokalemia and a potassium preparation and the like can be encapsulated together. Furthermore, by encapsulating an oral taking drug having poor absorptivity and the like and an ingredient having an effect of promoting the absorption thereof and the like together, the edible film packaging 1 which aims to promote absorption can be also obtained. For example, an iron preparation and vitamin C which promotes the absorption thereof can be encapsulated together.

The edible film packaging 1 described above is not limited to a single-layered structure, and can be designed to a two or more-layered structure according to adhesion properties and the intended use.

It should be noted that the edible film packaging 1. in which the oral material 2 has been encapsulated beforehand is marketed, as it is, as an individual package. In the conventional case where a taking assist product, such as wafer paper and a taking assist jelly, is used at the time of taking or having-or-swallowing oral taking drugs or quasi drugs, functional food, etc., differences in taking properties and ease of having-or-swallowing sometimes arise depending on a use manner of a user. However, since the edible film packaging 1 is marketed while the oral material 2 being encapsulating beforehand, differences in taking properties and ease of having-or-swallowing among users are difficult to arise. Moreover, a user does not need to go to a drug store or the like to purchase a taking assist product in each use. Furthermore, a user does not need to prepare a preparation using a taking assist product and oral taking drugs or quasi drugs, foodstuffs, etc., in each intake, thereby eliminating troublesomeness.

In a marketing stage, in order to prevent the edible film packaging 1 from damaging upon contact with moisture or the like and to ensure the quality of the oral material 2, outside packaging can be further provided to the edible film packaging 1 at will. The outside packaging is not narrowly limited, and for example, a pillow packaging form, a bottle packaging form, etc., using an appropriate film are mentioned.

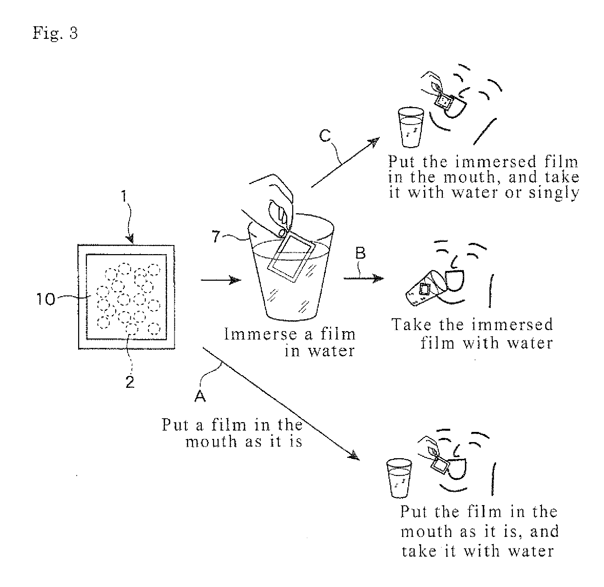

Fig. 3 is a view for describing a method of taking or having-or-swallowing the edible film packaging 1 according to the present invention.

The first taking or having-or-swallowing method is an example of putting the edible film packaging 1 in the mouth as it is, and holding water in the mouth for taking or having-or-swallowing the edible film packaging 1 with water (see Fig. 3A). Since the edible film packaging 1 according to the present invention is softened upon contact with saliva or water and is deformed, swallowing without any unpleasantness feeling can be performed.

The second taking or having-or-swallowing method is an example of immersing the edible film packaging 1 in water contained in a container 7, such as a glass, for a given period of time (e.g., for several seconds) for softening, and then pouring the edible film packaging 1 into the mouth with water for taking or having-or-sw allowing (see Fig. 3B). The edible film packaging according to the present invention has moderate strength against water. Therefore, even when the edible film packaging according to the present invention is immersed in water for a given period of time, it is not broken and can be softened. Especially for elderly persons who have reduced amount of salivation, the above method is an effective taking or having-or-swallowing method.

The third taking or having-or-swallowing method is an example of immersing the edible film packaging 1 in water contained in a container 7, such as a glass, for a given period of time (e.g., for several seconds) for softening, once taking the softened edible film packaging 1 out of water, putting the same in the mouth as it is, and then taking or having-or-swallowing the same with water or singly (see Fig. 3C). The edible film packaging can be softened with water without being broken similarly as in the second taking or having-or-swallowing method. This method is an effective taking or having-or-swallowing method particularly for patients who are limited in a liquid intake amount.

The above-described taking or having-or-swallowing methods A to C effectively utilize properties that the edible film packaging 1 according to the present invention is softened upon contact with saliva or water, and is easy to pass through the throat and esophagus. Depending on the kind of the edible film material 10, the sealed part 11 of the edible film packaging 1 is sometimes hardened as compared with unsealed parts. In this case, the taking or having- or- swallowing methods (B, C) described above are more useful which have been devised in such a manner as to put, in the mouth, the edible film packaging whose sealed part 11 has been moderately softened beforehand by immersing the edible film packaging 1 in water before taking or having-or-swallowing. It should be noted that when the size of the edible film packaging 1 is small, a taking or having- or- swallowing method is also employable which involves putting the edible film packaging 1 in the mouth to be brought into contact with saliva for softening the edible film packaging 1, and then swallowing the same, as it is.

### Examples

### Example 1

In Example 1, time for each edible film material to reach equilibrium moisture was measured since an edible film which has reached equilibrium moisture is used for evaluating more accurate physical properties in Examples 3 to 5 mentioned later.

About 500 mg of each of a starch film (manufactured by Tsukioka Co., Ltd., the same applies in the following description), a gelatin film (manufactured by Tsukioka Co., Ltd., the same applies in the following description), a casein film (manufactured by Tsukioka Co., Ltd., the same applies in the following description), a pullulan film (Manufactured by HAYASHIBARA SHOJI, INC., the same applies in the following description), and an alginic acid film (Ina Food Industry Co., Ltd., the same applies in the following description) was put in a plastic petri dish, and stored in a storage room to measure the variation in mass. The conditions of the storage room were controlled to long term storage test conditions of a temperature of 25C ± 2°C and a relative humidity of 60%RH ± 5% RH, which are specified in the ICH guideline as stability test conditions for drugs.

The results are shown in table 1. As shown in Table 1, when the edible film material was stored for two or more days in the atmosphere of a temperature of 25°C ± 2°C and a relative humidity of 60%RH ± 5% RH, the moisture of the edible film material reached equilibrium and the mass became constant irrespective of the type of the edible film material.

**[Table 1]**

| Elapsed time after storage | Variation in mass of edible film | | | | |
|---|---|---|---|---|---|
| | Starch film | Gelatin film | Casein film | Pullulan film | Alginic acid film |
| Just before starting | 501mg | 503mg | 498mg | 502mg | 499mg |
| 8hours later | 518mg | 507mg | 502mg | 555mg | 510mg |
| 24hours later | 523mg | 508mg | 503mg | 560mg | 515g |
| 48hours later | 526mg | 511mg | 504mg | 576mg | 518g |
| 72hours later | 525mg | 511mg | 504mg | 576mg | 517mg |
| 96hours later | 526mg | 511mg | 504mg | 575mg | 518mg |

Example 1 showed that when the edible film material is stored for two or more days in the atmosphere of a temperature of 25°C ± 2°C and a relative humidity of 60%RH ± 5% RH, the edible film material reaches equilibrium moisture irrespective of the type of the film.

### Example 2

In Example 2, a specific moisture amount of each edible film material at the time of equilibrium moisture was measured since an edible film which has reached equilibrium moisture is used for evaluating more accurate physical properties in Examples 3 to 5 mentioned later.

First, about 500 mg of each edible film material was put in a plastic petri dish, and stored in the same condition as in Example 1 (a temperature of 25°C ± 2°C and a relative humidity of 60°/RH ± 5% RH). Then, the equilibrium moisture of the edible film material was measured before storage and after 48 hours of storage.

Specifically, the moisture was measured as follows. First, about 0.1g or more of each edible film material sample was put on a scale pan of a heating and drying moisture analyzer (manufactured by A&D Company Limited, Model: MX-50, Registration number: P1002781), and the temperature was increased to 130°C. In this state, the mass of the edible film material sample was measured at intervals of 1 minute. A point where the variation in mass at each measurement point was 0.1% or lower was defined as an end point, and the moisture content, at the time when equilibrium moisture was achieved, was calculated from the reduction value of the mass.

The results are shown in Table 2. It was revealed that the equilibrium moisture at a temperature of 25°C ± 2°C and a relative humidity of 60%RH ± 5% RH of each of the starch film, the gelatin film, the casein film, the pullulan film, and the alginic acid film was 11.28%, 13.70%, 12.06%, 27.43%, and 24.50%, respectively.

**[Table 2]**

| Elapsed time after storage | Equilibrium moisture amount of edible film (%) | | | | |
|---|---|---|---|---|---|
| | Starch film | Gelatin film | Casein film | Pullulan film | Alginic acid film |
| Just before starting | 6.83 | 12.44 | 11.03 | 16.77 | 21.52 |
| 48hours later | 11.28 | 13.70 | 12.06 | 27.43 | 24.50 |

### Example 3

In Example 3, flexibility (ease of deformation) of each edible film material was evaluated.

Flexibility (ease of deformation) evaluation was performed for a starch film having a thickness of about 10 µm (hereinafter referred to as a "10 µm starch film"), a gelatin film having a thickness of about 10 µm (hereinafter referred to as a "10 µm gelatin film"), a gelatin film having a thickness of about 20 µm (hereinafter referred to as a "20 µm gelatin film"), a gelatin film having a thickness of about 40 µm (hereinafter referred to as a "40 µm gelatin films"), a casein film having a thickness of about 30 µm (hereinafter referred to as a "30 µm casein film"), a casein film having a thickness of about 50 µm (hereinafter referred to as a on µm casein films"), a casein film having a thickness of about 80 µm (hereinafter referred to as a "80 µm casein film"), a pullulan film having a thickness of about 20 µm (hereinafter referred to as a "20 µm pullulan film"), a pullulan film having a thickness of about 30 µm (hereinafter referred to as a "30 µm pullulan film"), and an alginic acid film having a thickness of about 30 µm (hereinafter referred to as a "30 µm alginic acid film").

First, under the same conditions as in Examples 1 and 2 (a temperature of 25°C ± 2°C and a relative humidity of 60%RH ± 5% RH), each edible film material was stored for two or more days to be adjusted to equilibrium moisture. Thereafter, flexibility (ease of deformation) was evaluated by the method described below under the same conditions (a temperature of 25°C ± 2°C and a relative humidity of 60%RH ± 5% RH).

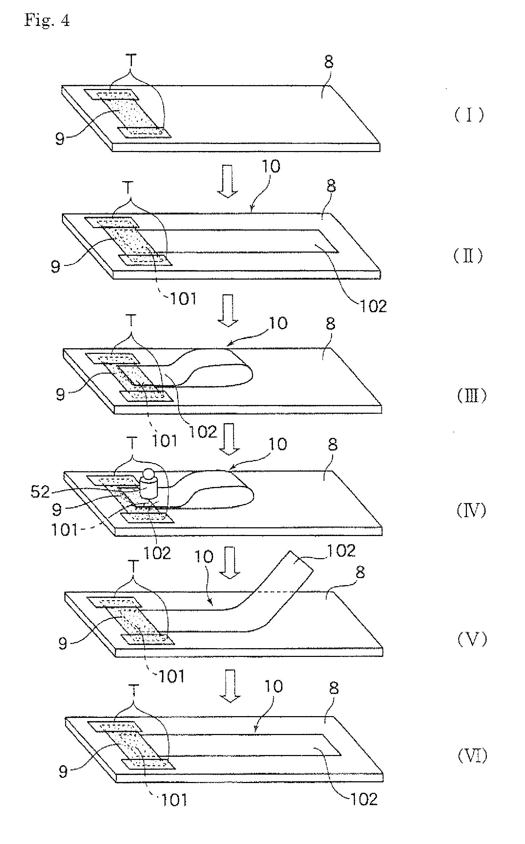

Each edible film material was evaluated for flexibility (ease of deformation) as follows. The evaluation method will be described with reference to Fig. 4. First, a 2 × 4 cm polyvinyl chloride film 9 having a thickness of about 250 µm was placed on an acrylic sample stage 8, and both ends of the polyvinyl chloride film 9 were fixed to the sample stage 8 with a cellophane tape T (see Fig. 4(I)). Next, one end 101 of each edible film material sample 10 cut out into a size of 2 cm × 10 cm was inserted between the sample stage 8 and the polyvinyl chloride film 9, and fixed (see Fig. 4 (II)). Then, the edible film material sample 10 was folded from an opposite end 102 of the edible film material sample 10 while producing a curvature without forming a crease in the edible film material sample 10 as shown in Fig. 4 (III). Then, the sample stage 8, the one end 101 of the edible film material sample 10, the polyvinyl chloride film 9, and the opposite end 102 of the edible film material sample 10 were laminated from a lower layer in the stated order. A10 g weight 52 was placed on a portion where each ends of the edible film material sample 10 were laminated (see Fig. 4 (IV)). About 30 seconds later, the weight 52 was removed. Then, a period of time from the removal of the weight 52 until the opposite end 102 of the edible film material sample 10 was returned to a state where the sample 10 was in contact with the sample stage 8 was measured (see Figs. 4(V) and (VI)).

It is considered that the edible film material 10 having longer measurement time has higher flexibility (ease of deformation). Therefore, the flexibility (ease of deformation) score was determined based on the measurement time according to the criteria shown in Table 3. It should be noted that the measurement was repeated three times for each edible film material 10, thereby determining the average flexibility (ease of deformation) score.

**[Table 3]**

| Measurement time | Flexibility (ease of deformation) score |
|---|---|
| Lower than 1 minute | 1 |
| 1 minute or more and lower than 2 minutes | 2 |
| 2 minutes or more and lower than 3 minutes | 3 |
| 3 minutes or more | 4 |

The results are shown in Table 4. In the case of the edible film materials 10 having a thickness lower than 50 µm, the measurement time of each film material was 3 minutes or more and the average flexibility (ease of deformation) score thereof was 4 points irrespective of the kind of the edible film material 10. On the contrary, in the case of the edible film materials 10 having a thickness of 50 µm or more, the measurement time of each film material was lower than 1 minute and the average flexibility (ease of deformation) score thereof was 1 point.

**[Table 4]**

| Designation of edible film | Measurement time | Flexibility (ease of deformation) score |
|---|---|---|
| 10µm Starch film | 3 minutes or more | 4 |
| 10µm Gelatin film | 3 minutes or more | 4 |
| 20µm Gelatin film | 3 minutes or more | 4 |
| 40µm Gelatin film | 3 minutes or more | 4 |
| 30µm Casein film | 3 minutes or more | 4 |
| 50µm Casein film | Lower than 1 minute (0 second 58) | 1 |
| 80µm Casein film | Lower than 1 minute (25 seconds 51) | 1 |
| 20µm Pullulan film | 3 minutes or more | 4 |
| 30µm Pullulan film | 3 minutes or more | 4 |
| 30µm Alginic acid film | 3 minutes or more | 4 |

Example 3 showed that in the edible film material 10 having a thickness lower than 50 µm, the flexibility (ease of deformation) is excellent irrespective of the kind of the edible film material 10. On the contrary, it was revealed that in the edible film material 10 having a thickness of 50 µm or more, the flexibility (ease of deformation) is poor.

### Example 4

In Example 4, strength against water of each edible film was evaluated.

All the edible film materials which were evaluated for flexibility in Example 3 were stored for two or more days under the same conditions as in Examples 1, 2, and 3 (a temperature of 25°C ± 2°C and a relative humidity of 60%RH ± 5% RH) to be adjusted to equilibrium moisture. The strength against water was evaluated by the method described below under the same conditions (a temperature of 25°C ± 2°C and a relative humidity of 60%RH ± 5% RH).

Each edible film material was evaluated for strength against water as follows. The evaluation method will be described with reference to Fig. 2. The edible film material 10 was placed on an opening part of a 10 mL beaker 3 (see Fig. 2(I)), and fixed with a rubber band 4 (see Fig. 2(II)). Then, a 1 g weight 51 was put on the center portion of the edible film material 10. 50µL of 25°C purified water was added using a micropipet 6 to a depressed part 100 of the edible film material 10 due to the 1 g weight 51. Then, a period of time from the edible film material 10 was broken until the weight 51 was dropped in the beaker 3 was measured with a stopwatch.

In view of the fact that the taking properties are deteriorated even when the strength is excessively low or high as described above, strength scores were determined based on the measurement time according to the criteria shown in Table 5. It should be noted that the measurement was repeated three times for each edible film material 10, thereby determining the average strength score against water.

**[Table 5]**

| Average score value of weight drop time | Strength score |
|---|---|
| Lower than 20 seconds or 10 minutes or more | 1 |
| 20 seconds or more and lower than 2 minutes | 2 |
| 2 minutes or more and lower than 4 minutes | 3 |
| 4 minutes or more and lower than 6 minutes | 4 |
| 6 minutes or more and lower than 8 minutes | 5 |
| 8 minutes or more and lower than 10 minutes | 6 |

The results are shown in Table 6. With respect to the 10 µm starch film, the weight 51 was dropped in only second after the addition of purified water. Moreover, with respect to the 40 µm gelatin film and the 80 µm casein films, the weight 51 was not dropped until 10 or more minutes had passed after the addition of purified water. With respect to each of the other edible film materials, the period of time until the weight. 51 was dropped from the addition of purified water was from about 2 minutes to about 8 minutes.

**[Table 6]**

| Designation of edible film | Average score value of weight drop time | Strength score |
|---|---|---|
| 10µm Starch film | 1 second | 1 |
| 10µm Gelatin film | 2 minutes 13 seconds | 3 |
| 20µm Gelatin film | 8 minutes 41 seconds | 6 |
| 40µm Gelatin film | 10 minutes or more | 1 |
| 30µmCasein film | 4 minutes 44 seconds | 4 |
| 50µm Casein film | 7 minutes 33 seconds | 5 |
| 80µm Casein film | 10 minutes or more | 1 |
| 20µm Pullulan film | 6 minutes 20 seconds | 5 |
| 30µm Pullulan film | 6 minutes 47 seconds | 5 |
| 30µm Alginic acid film | 4 minutes 46 seconds | 4 |

From the results of Example 4, it is considered that since the strength against water of the 10 µm starch film is extremely low, it is difficult to use the 10 µm starch film as a material of the edible film packaging 1 according to the present invention. Generally, the 10 µm starch film is formed into wafer paper. In contrast, the strength against water of each of the 40 µm gelatin film and the 80 µm casein film is excessively high, and thus release of the contents of the edible film packaging 1 is prevented. Thus, such films are not preferable. It should be noted that the gelatin films showed a tendency to have higher strength as compared with other films.

It is considered that the flexibility (deformation ease) and the strength against water of the edible film material sample 10 have influence on the quality of the taking properties of the edible film packaging 1. In view of the above, the total of the flexibility (deformation ease) score obtained in Example 3 and the strength score obtained in Example 4 was defined as a physical property score indicating the overall evaluation of physical properties. The physical property scores are shown in Table 7.

**[Table 7]**

| Designation of edible film | Flexibility (ease of deformation) score | Strength score | Physical property score |
|---|---|---|---|
| 10µm Starch film | 4 | 1 | 5 |
| 10µm Gelatin film | 4 | 3 | 7 |
| 20µm Gelatin film | 4 | 6 | 10 |
| 40µm Gelatin film | 4 | 1 | 5 |
| 30µm Casein film, | 4 | 4 | 8 |
| 50µm Casein film | 1 | 5 | 6 |
| 80µm Casein film | 1 | 1 | 2 |
| 20µm Pullulan film | 4 | 5 | 9 |
| 30µm Pullulan film | 4 | 5 | 9 |
| 30µm Alginic acid film | 4 | 4 | 8 |

### Example 5

In Example 5, it was analyzed whether there is a correlation between the actual taking properties of the edible film packaging 1 and the scores of the physical properties of the edible film material sample 10 obtained above.

### <production of edible film packaging 1>

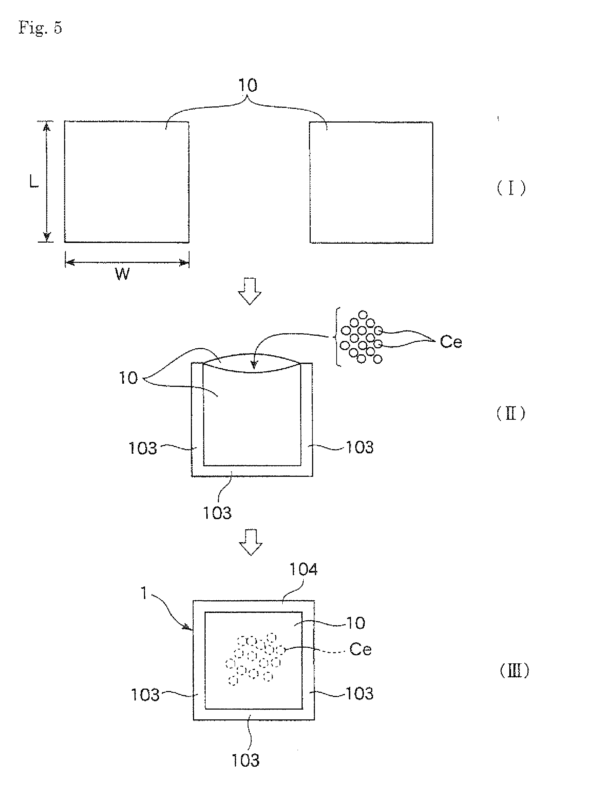

The edible film packaging 1 was produced as follows. The production method will be described with reference to Fig. 5. Each edible film material 10 used in Examples 3 and 4 were cut out into a size of 30mm cm in width W, 30mm in length L to obtain two pieces (see Fig. 5 (I)). The cut out pieces of the edible film material 10 were laminated, and three sides 103 were heat sealed to produce a bag using the edible film material 10. About 1 g of crystalline cellulose Ce was put therein (see Tig. 5 (II)). Then, a one side 104 of an opening part was heat sealed to produce the edible film packaging 1.

### <Evaluation of taking properties of edible film packaging 1>

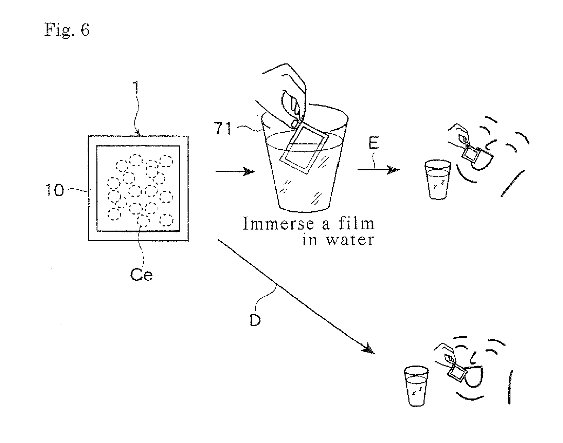

The edible film packaging 1 obtained above was given to three volunteers by the following two methods. The taking methods will be described with reference to Fig. 6. First, a method of putting the edible film packaging 1 in the mouth, and taking the same with water was defined as a taking method D. A method of holding one end of the edible film packaging 1 by the hand, immersing the edible film packaging 1 in water in a glass 71 for about 5 seconds, taking the edible film packaging 1 out of the glass after immersion, putting the edible film packaging 1 in the mouth, and taking the edible film packaging 1 with water was defined as a taking method E. It should be noted that, at the time of taking, the taking order was randomized so that the designation of the edible film packaging 1, the material of the edible film, etc., were not recognized.

With respect to the taking properties, the scores of the taking properties were obtained according to the evaluation criteria for the taking properties of Table 8. Here, "Intakable" in Table 8 refers to, for example, a case where the edible film packaging 1 was not softened and not deformed in the mouth, resulting in a failure of swallowing the edible film packaging 1 and a case where the edible film 7 of the surface of the edible film packaging 1 was broken before taking, and thus crystalline cellulose Ce as the contents jumped out of the edible packaging 1.

**[Table 8]**

| Taking level | Taking score |
|---|---|
| Very easy to take | 6 |
| Easy to take | 5 |
| Takable | 4 |
| Difficult to take | 3 |
| Very Difficult to take | 2 |
| Intakable | 1 |

### <Overall judgment of taking properties of edible film packaging 1>

From the scores of the taking properties obtained by the three volunteers, the total average value at the time of each taking was calculated. Then, the overall judgment of the taking properties of the edible film packaging 1 at the time of each taking was carried out according to the criteria shown in Table 9. With respect to the taking properties of the edible film packaging 1, the range of the total average score of the taking properties of 5.0 points or more and lower than 6.0 points was defined as "more useful (O)", the range of 3.0 points or more and lower than 5.0 points was defined as "Useful (Δ)", and the range of 1.0 point or more and lower than 3.0 points was defined as "Unuseful (X)". Then, the overall judgment of the taking properties was performed.

**[Table 9]**

| Total average score of taking properties | Overall judgment |
|---|---|
| 5.0 points or more and 6.0 points | O |
| 3.0 points or more and lower than 5.0 points | Δ |
| 1.0 point or more and lower than 3.0 points | X |

The results of the overall judgment of the taking properties are shown in Table 10. The 10 µm thick edible film packaging 1 showed relatively favorable taking properties according to the taking method D irrespective of the film raw material. According to the taking method E, however, there was a case where the outside edible film material 10 dissolved simply by immersing the edible film packaging 1 in water, and crystalline cellulose Ce as the contents were released in water, resulting in a failure of taking. In the overall judgment, the 10 µm thick edible film packaging 1 was judged to be useful (Δ).

With respect to the edible film packaging 1 using gelatin or casein, there are slight differences in the taking properties according to the taking method when the film thickness ranges from 20 to 50 µm. The edible film packaging 1 using gelatin or casein was judged to be useful (Δ). In contrast, with respect to the edible film packaging 1 using casein and having a thickness of 80 µm, the taking properties were poor according to the taking method D because the edible film material 10 was too hard. Also according to the taking method E, although the edible film material 10 was somewhat softened by being immersed in water, the taking properties were poor. In the overall judgment, the edible film packaging 1 using casein and having a thickness of 80 µm was judged to be unuseful (X).

The edible film packaging 1 using pullulan or alginic acid was judged to be more useful (O) according to both the methods D and E in the overall judgment.

**[Table 10]**

| Edible film packaging | Taking score | | | | | | Total average | Overall judgment of taking properties |
|---|---|---|---|---|---|---|---|---|
| | Evaluator A | | Evaluator B | | Evaluator C | | | |
| | Taking method D | Taking method E | Taking method D | Taking method E | Taking method. D | Taking method E | | |
| 10µm Starch film packaging | 6 | 1 | 6 | 1 | 3 | 1 | 3.0 | Δ |
| 10µm Gelatin film packaging | 4 | 1 | 6 | 1 | 5 | 1 | 3.0 | Δ |
| 20µm Gelatin film packaging | 6 | 6 | 3 | 5 | 4 | 4 | 4.7 | Δ |
| 40µm Gelatin film packaging | 2 | 5 | 1 | 5 | 2 | 5 | 3.3 | Δ |
| 30µm Casein film packaging | 5 | 5 | 3 | 5 | 4 | 6 | 4.7 | Δ |
| 50µm Casein film packaging | 2 | 4 | 2 | 4 | 2 | 4 | 3.0 | Δ |
| 80µm Casein film packaging | 1 | 2 | 1 | 1 | 1 | 2 | 1.3 | X |
| 20µm Pullulan film packaging | 6 | 6 | 5 | 6 | 6 | 6 | 5.8 | O |
| 30µm Pullulan film packaging | 5 | 6 | 5 | 6 | 6 | 6 | 5.7 | O |
| 30µm Alginic acid film packaging | 5 | 5 | 5 | 5 | 5 | 5 | 5.0 | O |

### <Correlation between overall judgment of taking properties and physical properties>

The correlation between the overall judgment of the taking properties of the edible film packaging 1 obtained above and the physical properties of the edible film material 10 obtained in each of Examples 3 and 4 was analyzed by comparing them. The comparison between the overall judgment of the taking properties of each edible film packaging 1 and the score of the physical properties of each edible film material 10 is shown in Table 11.

**[Table 11]**

| Edible film packaging | Physical property score | Overall judgment of taking properties |
|---|---|---|
| 10µm Starch film packaging | 5 | Δ |
| 10µm Gelatin film packaging | 7 | Δ |
| 20µm Gelatin film packaging | 10 | Δ |
| 40µm Gelatin film packaging | 5 | Δ |
| 30µm Casein film packaging | 8 | Δ |
| 50µm Casein film packaging | 6 | Δ |
| 80µm Casein film packaging | 2 | X |
| 20µm Pullulan film packaging | 9 | O |
| 30µm Pullulan film packaging | 9 | O |
| 30µm Alginic acid film packaging | 8 | O |

The taking properties of each edible film packaging 1 produced using the edible film material 10 having scores of the physical properties of 5 points or more were all useful (Δ) or more useful (O) in the overall judgment. It was recognized that, as the physical properties required to have the overall judgment of "useful (Δ)" or "more useful (O)" in terms of the taking properties of the edible film material 10 used for the edible film packaging1, both the flexibility (ease of deformation) shown in Example 3 and the strength against water shown in Example 4 are mentioned.

Example 5 showed that when the scores of the physical properties of the edible film material 10 to be used are higher, the overall judgment of the taking properties of the edible film packaging 1 is more useful. That is, it was revealed that there is a correlation between the favorable actual taking properties of the edible film packaging 1 and the physical properties of the edible film material 10.

Moreover, the overall judgment of the flexibility (ease of deformation) analyzed in Example 3, the strength against water analyzed in Example 4, and the taking properties in each taking method analyzed in Example 5 revealed that the thickness of the edible film material 10 used for the edible film packaging 1 according to the present invention is preferably 20 to 50 µm. With respect to a suitable thickness for each edible film material, it was revealed that a gelatin film has a thickness of preferably lower than 40 µm and a casein film has a thickness of preferably lower than 80 µm and more preferably lower than 50 µm, for example.

### Example 6

In Example 6, it was analyzed for differences in the taking properties among: the case where the target oral material is taken in the form of the edible film packaging; the case where the target oral material is taken in a generally employed form; and the case where the target oral material is taken using a taking assist product. As the oral material, crystalline cellulose was used similarly as in Example 6.

### <Production of each pharmaceutical form>

As the edible film packaging, using a 30 µm alginic acid film as the edible film material 10, an edible film packaging containing 2 g of crystalline cellulose was produced in the same manner as in Example 5. As a generally employed pharmaceutical form, subtle granules in which 2 g of crystalline cellulose is used as it is (hereinafter referred to as "subtle granules") and a capsule agent in which 2 g of crystalline cellulose was portioned by 400 mg into each of five No. 1 gelatin capsules, and charged therein (hereinafter referred to as a "capsule agent") were produced. As a case where a taking assist product is used, a substance in which 2 g of crystalline cellulose was put in a commercially-available bag-like wafer paper (hereinafter referred to as a "bag-like wafer paper preparation") and a substance in which 2 g of crystalline cellulose was mixed in a commercially-available taking assist jelly (hereinafter referred to as a "jelly preparation") were produced and used.

### <Evaluation of taking properties>

27 volunteers evaluated taking properties using 5 kinds of pharmaceutical forms produced above. The five kinds of pharmaceutical forms were evaluated and scored according to the criteria for the taking properties shown in Table 12. Simultaneously therewith, the five kinds of pharmaceutical forms were ranked in terms of ease of taking, and scored according to Table 13.

**[Table 12]**

| Taking level | Score |
|---|---|
| Very easy to take | 6 |
| Easy to take | 5 |
| Takable | 4 |
| Difficult to take | 3 |
| Very Difficult to take | 2 |
| Intakable | 1 |

**[Table 13]**

| Ranking | Score |
|---|---|
| First place | 5 |
| Second place | 4 |
| Third place | 3 |
| Fourth place | 2 |
| Fifth place | 1 |

The average score of each evaluation result and the total thereof are shown in Table 14. In the comparison of the average score of the taking properties, the edible film packaging, the subtle granules, the capsule agent, the bag-like wafer paper preparation, and the jelly preparation are ranked in the stated order, which revealed that the taking properties of the edible film packaging of the present invention is the most favorable among the five kinds of the pharmaceutical forms. In the comparison of the average score value of ranking of the taking properties, the edible film packaging of the present invention, the subtle granules, the capsule agent, the bag-like wafer paper preparation, and the jelly preparation are ranked in the stated order, which revealed that the taking properties of the edible film packaging of the present invention is the most favorable among the five kinds of the pharmaceutical forms. Moreover, it was revealed that, also with respect to the total of the evaluation scores of the taking properties and the ranking scores of the taking properties, the edible film packaging of the present invention is the most favorable.

Example 6 showed that taking properties in the case of taking the target oral material in the form of the edible film packaging is more excellent than in the case of taking the target oral material in a generally employed pharmaceutical form and in the case of taking the target oral material using a taking assist product.

**[Table 14]**

| Pharmaceutical form | Average evaluation score value of taking properties | Average ranking score value of taking properties | Total score |
|---|---|---|---|
| Edible film packaging material | 4.4 | 3.8 | 8.2 |
| Bag-like wafer paper preparation | 3.6 | 2.0 | 5.6 |
| Subtle granules | 4.1 | 3.4 | 7.5 |
| Capsule agent | 4.1 | 3.0 | 7.1 |
| Jelly agent | 3.6 | 1.7 | 5.3 |

### Industrial Applicability

The edible film packaging according to the present invention can be utilized as a novel packaging in which oral materials, such as oral taking drugs or quasi drugs, or foodstuffs, are encapsulated and which can be taken or had-or-swallowed as it is. The edible film packaging can be used as a novel pharmaceutical form which does not fall under the category of the conventional capsule agents; tablets; powder, subtle granules, and pellets (so-called powdered medicine); jelly agents; and liquid agents. Moreover, the edible film packaging can be utilized as a novel having-or-swallowing form for foodstuffs, such as functional foods having a recommended suitable intake amount.

### Brief Description of the Drawings

Fig. 1 is a view showing one embodiment of an edible film packaging 1 according to the present invention.
Fig. 2 is a view illustrating a method of evaluating the strength against water of each edible film material 10.
Fig. 3 is a view for describing a taking or having- or- swallowing method of the edible film packaging 1 according to the present invention.
Fig. 4 is a view illustrating a method of evaluating the flexibility of each edible film material 10 in Example 3.
Fig. 5 is a view illustrating a method of producing the edible film packaging in Example 5.
Fig. 6 is a view illustrating a method of taking the edible film packaging 1 in Example 5.

### Description of Reference Numerals

- 1.: Edible film packaging
- 2.: Oral material
- 11.: Sealed part
- 10.: Edible film material (sample)
- 100.: Depressed part
- 3.: 10 mL beaker
- 4.: Rubber band
- 51.: 1 g weight
- 6.: Micropipet
- 7.: Container
- A, B, C.: Taking or having-or-swallowing method
- 8.: Sample stage
- 9.: Polyvinyl chloride film
- T.: Cellophane tape
- 101.: One end of edible film material sample
- 102.: Opposite end of edible film material sample
- 52.: 10 g weight
- Ce.: Crystalline cellulose
- 71.: Glass
- D, E.: Taking method

## Claims

1. An edible film packaging:
comprising an edible film material which has adhesion properties and is softened upon contact with moisture, and
the edible film packaging being taken or had-or-swallowed while an oral material being encapsulated.

2. The edible film packaging according to claim 1, produced utilizing thermal adhesion properties.

3. The edible film packaging according to claim 1, comprising an edible film material having a thickness of 20 to 50 µm.

4. The edible film packaging according to claim 1, comprising the edible film material, wherein the edible film material is placed on an opening part of a 10 mL beaker specified in Japanese Industrial Standard (JIS standard) R3503; the edible film material is fixed with a rubber band in such a manner as to cover the opening part of the beaker; then a 1 g weight specified in Japanese Industrial Standard (JIS standard) B7609 is put on the center portion of the edible film material; and 50 µL of 25°C purified water is added to a depressed part of the edible film material due to the 1 g weight, a period of time from the edible film material is broken until the 1 g weight is dropped in the 10 mL beaker is 2 minutes or more and lower than 10 minutes.

5. The edible film packaging according to claim 1, wherein, as the oral material, an oral taking drug or quasi drug is used.

6. The edible film packaging according to claim 1, wherein, as the oral material, a foodstuff is used.

7. An oral-material taking method, comprising taking or having-or-swallowing the edible film packaging according to any one of claims 1 to 6 as it is without opening.

8. An oral-material taking method, comprising taking or having-or-swallowing the edible film packaging according to any one of claims 1 to 6 as it is without opening after being immersed in water for a given period of time.
